Europäisches Patentamt

European Patent Office

Office européen des brevets

(11) Publication number: **0 414 130 A1**

# EUROPEAN PATENT APPLICATION

(21) Application number: **90115695.0**

(22) Date of filing: **16.08.90**

(51) Int. Cl.⁵: **A61B 19/00**

(30) Priority: **16.08.89 US 394564**

(43) Date of publication of application:
**27.02.91 Bulletin 91/09**

(84) Designated Contracting States:
**AT BE CH DE DK ES FR GB GR IT LI LU NL SE**

(71) Applicant: **Cosman, Eric Richard**
**872 Concord Avenue**
**Belmont Massachusetts 021 78(US)**

Applicant: **Onik, Gary Mark**
**263 Courtney Place**
**Wexford, Penn. 15090(US)**

(72) Inventor: **Cosman, Eric Richard**
**872 Concord Avenue**
**Belmont Massachusetts 021 78(US)**
Inventor: **Onik, Gary Mark**
**263 Courtney Place**
**Wexford, Penn. 15090(US)**

(74) Representative: **Eisenführ, Speiser & Strasse**
**Zweibrückenstrasse 17**
**D-8000 München 2(DE)**

(54) **A hand-held body stereotactic instrument.**

(57) This invention relates to a hand-held stereotactic instrument which enables quantitative direction of a needle or probe into the body. The instrument has a protractor type angle dial (3, 2003) which enables angulation of a probe relative to a gravitational reference orientation (20). The establishment of the gravitational vertical line for example is done by a simple level device such as a bubble level (2). Other alignment means on the instrument enables alignment to other axes of the body which may be related to x-ray or CT or MRI tomographic scanning axes. In its use, data is taken from tomographic CT scans or x-rays, and converted to an appropriate angle which is set on the instrument's angle dial. This in turn can be used to direct a probe at a desired angle into the body. The instrument can be used in connection with reference skin localizers to establish an entry point. Fulcrum or body contact means enable stabilization of the device near the entry point, eliminating translation movement variations, problems of weight of the device, and reducing the degree of freedom needed to hold the probe direction.

Fig. 11

# A HAND-HELD BODY STEREOTACTIC INSTRUMENT

Drs. Cosman and Onik have published several articles on the use of a stereotactic system to accurately direct a probe into a body based on CT tomographic image data. The method and instrumentation used are also disclosed in the Patent by Cosman, Onik and Wells in 1986, Patent No. 4,583,538. In that patent and the articles, a method is described which makes use of a skin localizer to establish a point on the surface of the patient's body which is related to an entry point seen on a CT tomographic image. Also seen in the CT image is a target point in depth in the body. From these two points, the entry point on the skin of the patient, and the target point, an angle of approach is established relative to the natural coordinate axes of the CT scanner machine. One of these axes is typically the gravitational vertical axis when the scan slice is vertical or non-tilted. In the method of Onik, Cosman and Wells, a stereotactic instrument which can hold a needle or other probe is so designed that it can establish a guide means that will line up in space according to the probe direction as calculated in the CT image. The instrument is then used to carry the probe tip to the entry point established on the patient's body as described above. Then, by pushing the probe into the body, the direction of the probe will correspond to the angles established from the CT scan. Thus, the method relies on a predetermined entry point plus angular approach means to direct the probe, in depth, to the desired target. The skin localizer, which has also been described in a patent application by Cosman and Onik, is a useful device to establish the desired entry point corresponding to the CT scan although the entry point may be determined in other ways. A variety of stereotactic apparatus can be devised to achieve the appropriate angular directions so as to align the probe shaft according to the desired probe path line established on the CT scan. Several such embodiments were described in the patent by Onik, Cosman and Wells. One of the problems with the invention described by Onik, Cosman, and Wells is that the needle holder is stabilized relative to the instrument which in one of their embodiments is attached to the floor or could be equally attached to the table. This means with respiratory variations in the skin of the patient while he is breathing or moving, that the needle holder would have differential variations relative to the entry point or the patient's skin where the entry point is located. This means inaccuracy in the path of the needle relative to the anatomy.

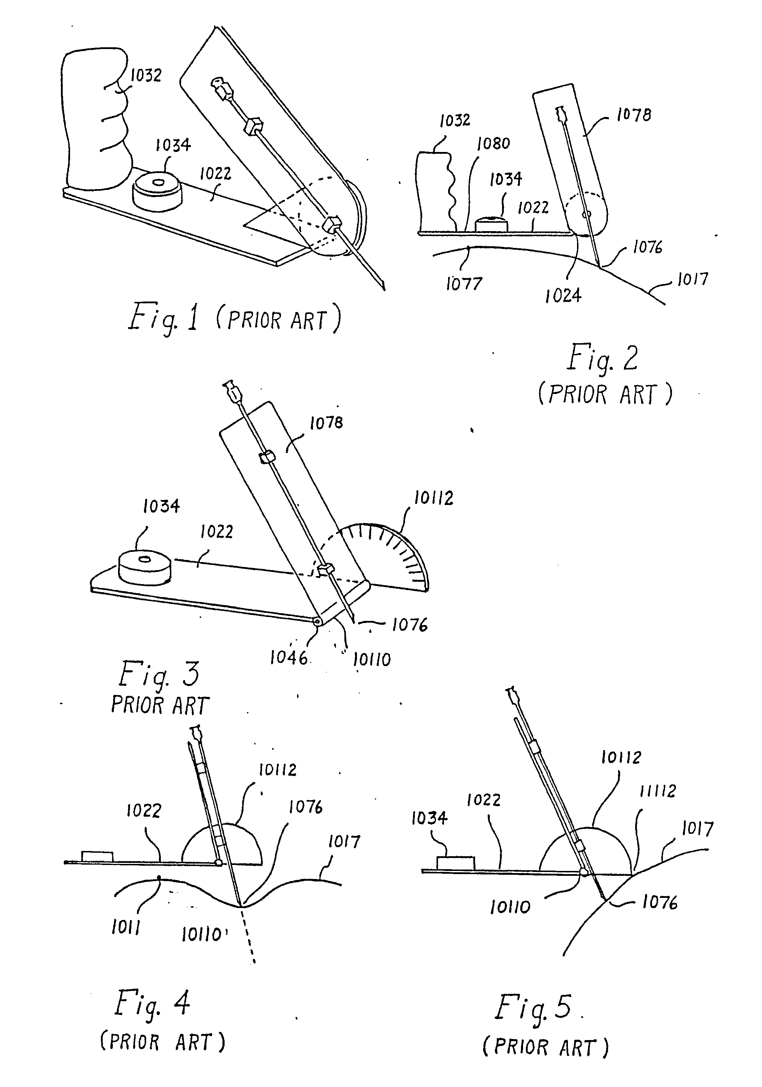

Figure 1 shows an invention of prior art which is taken from U.S. Patent Number 4,733,661 by Palestrant, issued March 29, 1988. It shows a hand-held, bubble-level stereotactic instrument for implanting surgical probes in the body. The teaching of that patent, all of its figures, and its claims indicate its use to be setting an angle with angular plate 1078 relative to horizontal plate 1022 and passing needle 1076 into the body. This instrument has several disadvantages for use as a hand-held device. In the patent there is no mention of contact of the instrument to the human body. Therefore it is necessary to hold the instrument in the air by handle 1032, pointing the needle tip 1076 to a desired point on the skin where you wish to pass the probe, stabilizing the instrument by means of bubble-level 1034 so that it remains horizontal, aligning the line 1094 in the plane of the CT scan, and then pushing the probe tip 1076 into the body. To do all this by hand the surgeon must stabilize the instrument in six degrees of freedom. That is, he must maintain the needle tip 1076 in translational degrees of freedom X, Y, Z so that it approximates the entry point. Those are three translational degrees of freedom which the surgeon must maintain. Furthermore, he must maintain alignment of reference 1094 to the scan plane. This is one additional angular degree of freedom. Finally, he must align the bubble-level 1034 in two angular directions about the gravitational horizontal (or equivalently the gravitational vertical line) to maintain the horizontal character of plate 1022 which is required for this instrument to work. In practice it is very difficult for a surgeon to be cognizant of all of these six degrees of freedom, stabilizing them simultaneously, while lining up the needle and passing the needle into the body. Thus the instrument of Figure 1 is impractical in the requirements it imposes on the surgeon.

A second deficiency of the invention of Figure 1 is that it does not allow for contact of the instrument to the patient's body in the vicinity of the entry point prior to passing the probe into the body. Indeed, nowhere in Patent 4,733,661 is it mentioned that this stereotactic instrument should be in contact with or stabilized by contact with the human body before passing the probe. Indeed, it is indicated that the stereotactic instrument is held in mid-air while the six degrees of freedom are stabilized as described above. It is clear from Figure 1 that there is no structure anticipated in that design to enable the contact of the instrument to the patient's skin near the point where the needle is passed into the skin. This is exemplified in Figure 2 which shows an elevation view of the device in Figure 1 in its use near the body. As described in the prior art patent, the arc slide 1078 is set at an angle relative to horizontal number 1022. Consider an axial view through the patient's body 107 where the slope is falling slightly away from the instrument as shown in Figure 2. Because platform 102 must be held horizontal by bubble-level 1034 while the patient holds the handle 1032, it must be that the point 1024 which is the closet point on the instrument near to the entry point or needle tip 1076 is still not in contact with the patient's body. Thus, with the design of Figure 1 as shown in Figure 2 is is impossible to have the instrument act as a pivot point around the entry point, that is it is impossible to have the instrument contact the entry point so that that contact point is a fulcrum for stabilizing the level of the instrument while still maintaining the horizontal number 1022 in the horizontal position.

Figure 3 shows another embodiment of the invention of the prior art patent 4,733,661. Here the angle member 1078 joins the horizontal member 1022 on a "piano hinge" bearing 10110. Also in that embodiment is angle dial 10112 which projects forward of the pivot 10110. It is clear from the structure in that figure that it also cannot be used according to the method of the present invention which will be described below, namely to contact the patient's skin at the entry point to stabilize the leveling and orientation of the instrument. The reason why it cannot be used in contact of the patient's skin and was not anticipated to be used in that way is illustrated in Figures 4 and 5. In Figure 4 we see a section of the patient's body 1017 which has an undulation in it. The entry point which is located at the tip of the needle 1076 is in the "valley" on the skin. To line up the instrument of Figure 3 which is shown in vertical elevation in Figure 4 and to maintain the horizontal portion 1022 level to gravity the instrument would have to be oriented as shown in Figure 4. This would mean that the distance from the pivot point 10110 to the entry point 1076 is considerable and it is impossible for the instrument near the probe guide or in the vicinity of the pivot point 10110 could contact the patient's skin anywhere near the entry point 1076. In fact, the nearest point where any pivoting could be done might be represented by the point 1011 and this is far away from the entry point 1076. Figure 5 reinforces the fact that the design of prior art in Figure 3 did not anticipate contacting the patient's body near the entry point. Notice in that figure that the patient's skin 107 falls away and under the instrument. If one were to attempt in this situation to bring the hinge point 10110 near to the entry point 1076, then the forward tip 11112 of the arc 10112 would hit the patient's body to prevent close proximity of the pivot point and guide tube to the entry point 1076 and still maintain the base plate 1022 in the horizontal position using the level 1034. Thus the designs shown for a hand-held stereotactic instrument in patent 4,733,661 have the deficiencies of not enabling the instrument to contact and stabilize against the patient's body prior to insertion of the needle into a chosen entry point. Not only would this contact provide stabilization and ease of horizontal leveling, but it would also have the important function of allowing the stereotactic instrument to ride along with the undulations and respiratory movements of the patient's body near the entry point. Because it cannot, there is further destabilization of the orientation of the stereotactic instrument relative to the body and the entry point and target which the instrument is designed to pass a probe through. Yet another deficiency of the design in Figure 1 and 3 is that the surgeon must support the entire weight of the instrument while he is attempting the maneuver of pushing the needle in and controlling the level and alignment as well as translational movement to track the body respirational movement. In fact in these designs he has to support the weight of the instrument by a handle on one end, meaning that there is not only the gravitational weight of the instrument but also the torque on his arm to maintain it in a horizontal alignment. This would be tiring and further complicate his ability to maintain the instrument in proper orientation.

Thus, an objective of the present invention is to provide a light-weight, hand-held stereotactic instrument which is portable and economical for doing biopsies on the body under CT localization.

Another objective of the present invention is to provide apparatus structure and methodology that the stereotactic instrument can contact the patient's skin in proximity to the predetermined entry point so as to stabilize the instrument in the process of aligning it. This contact or fulcrum stabilization will eliminate at least three translational degrees of freedom and one angular degree of freedom making it far easier for the surgeon to align the instrument to gravity.

Another objective of this invention is to enable the instrument to contact the patient's body so that it can ride along with the movements of the patient's body created by respiration or simple bodily movements. In this way the surgeon is freed from having to move the instrument independently to follow such bodily movements and it also eliminates the problems of machine or floor-fixed instruments which have no way of following such movements simply.

Yet another objective of the present invention is to have the instrument rest on the patient's body by contact means so that the weight of the instrument is relieved from the surgeon's hand, thereby further making it easy for him to stabilize and control the position of the instrument.

A further of the present invention is to have the instrument stabilized against the patient's body to eliminate variations of the approach caused by shaking or movement of the surgeon's hand.

Yet another objective of the present invention is to eliminate the need for a horizontal extended platform which must be leveled horizontal to gravity and held in free space to achieve accurate probe alignment to the target.

BRIEF DESCRIPTION OF THE PRESENT INVENTION

The present invention relates to a hand-held stereotactic device and method of use, but claims significant practicable improvements over the prior art. Specifically, it relates to an embodiment of a hand-held stereotactic device which is aligned relative to a gravitation reference or other CT machine reference, so as to direct the probe through a predetermined entry point to the target. A variety of means are described to align the apparatus manually to the vertical gravitation line and a significant one is the single spherical bubble level. Several other means are shown for aligning the other axes of the stereotactic instrument to the CT scanner plane or to an orthogonal line to the axial plane. The present invention has the advantages of being small and hand-held, and it does not require any horizontal base platforms which are large and impractical. A significant improvement in the present invention, furthermore, is that it has fulcrum or stabilizing means that specifically will contact the patient's skin near the predetermined entry point for the surgical probe without the rest of the instrument touching the body for a variety of body shapes. Thus, the instrument can be set against the patient's skin by this fulcrum means, and thereby all translational degrees of freedom of that point of the instrument are "locked onto", or removed as sources of inaccuracy, when the patient's body moves under respiration. In this way the present invention totally eliminates the ambiguities of translational variations of the stereotactic instrument relative to the patient's body, or the need for the surgeon to track these variations as he is trying to concentrate on inserting the probe into the body. A second objective of this present invention is to provide reference means near the probe holder and the position where the instrument contacts the body to orient the instrument so that the probe will pass into the plane of the CT scan. This feature is very important to remove one angular degree of freedom that the surgeon does not have to worry about in accurately placing the probe. Yet another advantage of the present invention is that it is intended to contact and rest against the skin near the entry point, and thus reduces a fraction of the weight of the instrument which the surgeon must hold up. This makes it easier for the surgeon to stabilize the instrument during the delicate process of both aligning the probe in the angular direction to hit the target as well as aligning the instrument to gravity.

DESCRIPTION OF THE DRAWINGS

Figure 1 shows one of the principle embodiments of prior art patent 4,733,661, this being a representation of Figure 1 of that prior patent.

Figure 2 shows prior art representing the instrument of Figure 1 used near the human body. This is a side view.

Figure 3 shows prior art and shows the other principle embodiment of the invention in U.S. Patent Number 4,733,661. This is a representation Figure 12 of that previous patent.

Figure 4 shows a side view of the prior art embodiment in Figure 3 being used relative to the human body for an uneven body surface.

Figure 5 shows a side view of the embodiment of the prior art instrument shown in Figure 3, also shown next to the human body where the surface of the body falls away from the instrument.

Figure 6 is an embodiment of the present invention which shows a protractor type vertical arc with slide and bubble level system to establish the device in line with the gravitational vertical. It also has an alignment and fulcrum edges near the entry point to enable alignment to the CT machine and stability against the skin.

Figure 7 shows a typical diagram of a CT slice with the skin localizer images in place and the vertical line and probe path to approach a target in depth of a body.

Figure 8 shows a side view using the instrument of the present invention shown in Figure 6 against the human body where the skin of the human body has an uneven contour similar to that illustrated in Figure 4.

Figure 9 shows another embodiment of the present invention which again has a vertical arc and different kind of probe guide with reference plane integral with the probe guide, and compact handle with bubble-level for holding the instrument vertical. It also illustrates the method of using such an instrument with a simple triangle-type skin localizer.

Figure 10 illustrates a CT scan, a sectional diagram of the human body showing the image points from the triangle localizer shown in Figure 9 as well as the method of determining the entry point from the localizer and determining the angle from the gravitational vertical to calculate the path to the target.

Figure 11 shows another embodiment to the present invention being held in the surgeon's hand, comprising a simple block structure with vertical angle dial and bubble-level together with a probe guide which is integral with the dial indicator.

Figure 12 shows a side view of the embodiment of Figure 11 with the probe guide approaching the skin of the patient.

Figure 13 shows the other side view of the embodiment of Figure 11 with the probe guide and needle in place at the patient's skin.

Figures 14a, 14b, and 14c show the utility of the embodiment of Figure 11 for various patient skin orientation and configuration.

Figure 15 shows how the embodiment of Figure 11 can be used for both axial and out of plane compound angular approaches.

Figure 16 shows a more elaborate embodiment of the device shown in Figure 1 with horizontal angular degrees of freedom in addition to the vertical arc degrees of freedom shown in Figure 1. This would be adapted for more complex, compound angle approaches.

## DESCRIPTION OF THE INVENTION

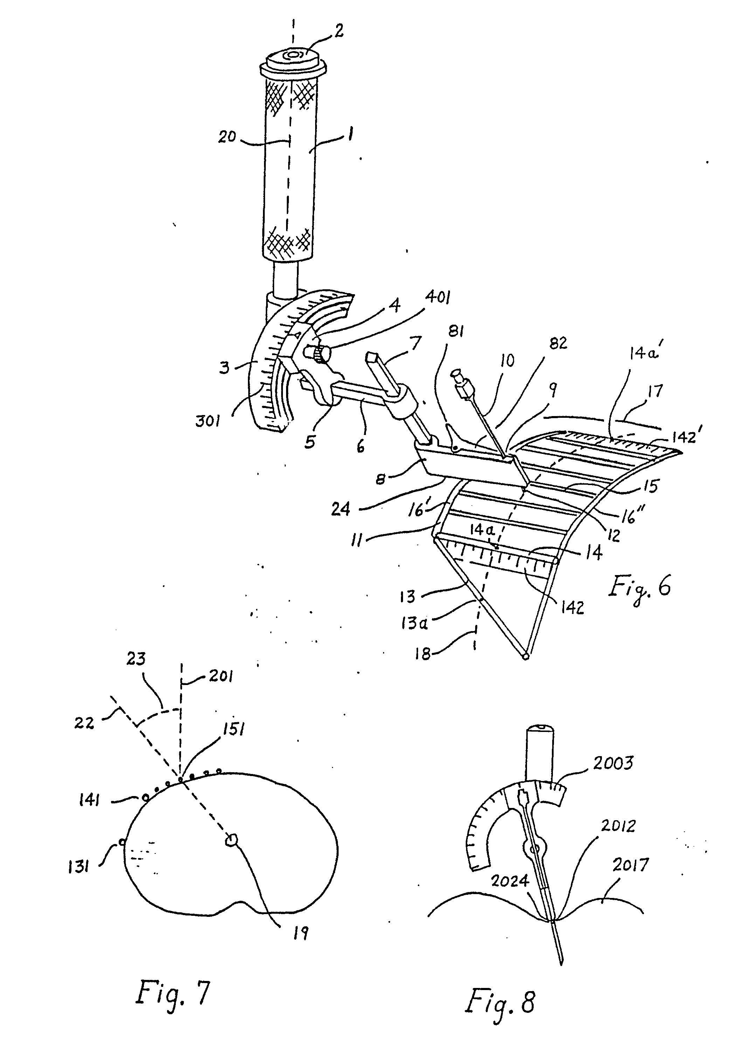

Figure 6 shows a typical embodiment of the present invention. The instrument can be hand-held by the surgeon with one hand by means of handle 1. The handle 1 has on its top a bubble level 2. This is a "spherical type" which enables with one bubble alignment of angles in two directions off of the vertical axis. By observing the level, the surgeon can easily maintain the axis of the handle 1 parallel to the gravitational vertical axis 20. The gravitational vertical axis means the natural vertical axis relative to gravity. On the bottom of handle 1 is attached the vertical arc 3, which has angular graduations on it. It is a vertical arc because the plane of the arc is perpendicular to the horizontal plane of the floor or Earth. The arc slide 4 can be oriented in any position by sliding along the arc 3, and can be clamped to it by a set screw or T-bolt 401. At the bottom of the arc slide is a square hole 5 in which square shaft 6 can slide. At the end of that shaft is another receptacle for square shaft 7, which also slides in it. At the base of square shaft 7 is the needle or probe clip holder 8. 8 has a movable side arm 82 actuated by the finger pressure pad 81, in such a way that the slot opening 9 will open up and accept a shaft illustrated by needle 10.

In operation, the stereotactic guide as shown in Figure 6 with a needle 10 in the clip holder can be brought into contact to the patient's body 17. On the patient's body is placed a "skin localizer" 11. This has been described in U.S. Patent No. 4,838,265 of Cosman and Onik. Many different types of skin localizers can be used, and a more simplified version was described in the patent by Onik, Cosman and Wells. The "skin localizer," in general, allows the surgeon to determine the "level" or position of the CT slice as it intersects the body 17. In the specific example shown in Figure 6, the localizer has on one end, a triangular section comprised of rods 13 and 14. These rods will appear as dots when intersected by a CT slice. An example of a CT slice image is shown in Figure 7, where the rod images are designated as 131 and 141, respectively. These spots are, in fact, the intersection of the CT plane with the triangle rods at the points 13A and 14A, in Figure 6. The path of the CT slice intersection along the patients body is designated by the dash curve 18 in Figure 6. Part of the specific localizer shown in Figure 6 are a series of parallel rods, for example rod 15, which are parallel to the right side 14 of the triangle and are attached together by two side runners 16′ and 16″.

As the CT slice intersects the body, these parallel rods will show up as spots in a sequence, as shown in Figure 7. Thus, from a CT image like that in Figure 7, one can determine from the measured distance on the slice between the images 131 and 141 and the known geometry of the triangle on the physical localizer in Figure 6, the distance along the scale 142 where the CT slice intersects that scale, i.e., the point 14a where the scan plane line 18 intersects the rod 14 of the triangle. With this information, the surgeon can project along a line over the patient's body, such as curve 18 in Figure 6, to determine where the CT slice intersects each of the parallel rods, such as rod 15 at point 12. Thus, by looking at Figure 7, one can select a desired parallel rod image point as an entry point on the surface of the patient's body. For example, on the rod designated as 15 in Figure 6, an entry point might be the point 12 corresponding to the image point 151 in Figure 7. That then might be an entry point through which the surgeon would wish to pass a biopsy needle to achieve target 19 in Figure 7.

The procedure for using the invention of Figure 6 with the corresponding CT scanner image of Figure 7 is illustrated as follows. From Figure 7 one would identify the vertical line 201 in the CT image which corresponds to the gravitational vertical on the actual patent. This vertical line 201 is determined directly from the CT console, since the CT machine is aligned relative to gravity, and such coordinate lines that can be brought up on the CT image at the CT console for all commonly available scanners. One would then determine the appropriate entry points along the skin localizer as shown by the series of dots in Figure 7. For this example, we have chosen image rod 151 as our entry point. The line 151 to 19 then defines the path of the needle approach 22. The CT console controls also enable us to determine directly the angle 23 between vertical 210 and probe path 22. One then sets the arc slide 4 in Figure 6 to the same angle 23 on the graduated arc 3. It is noted that the skin localizer 11 has been placed on the patient's skin in such a way that the side runner 16' and 16'' run essentially parallel to the axial CT Scan slice planes. This is easily arranged by lining them up using the laser lights of the CT scanner, for example. This means also that rods 15 and the rod 14 of the triangle, which are perpendicular to the side runners 16' and 16'' are essentially perpendicular to the CT scan plane when the scan plane is vertical and the patient's body does not fall off too steeply along the localizer. Now the needle 10 can be put into the needle guide hole 9. The bottom edge 24 of the clip holder 8 can be brought into parallel alignment with rod 15 such that the tip of the needle, emerging from the bottom of the clip holder hole 9, just approximates the point on the skin 12 corresponding to the desired entry point 151 in Figure 7. As described above, the entry point position 12 on rod 15 has been predetermined by calculating the "level" of the CT scan slice using the localizer data in Figure 7. Line 18 can be marked on the skin by a pen. It can be determined by the CT laser light line when the light is set through point 14a, or it can be drawn by projecting a line through point 14a on scale 142 and the corresponding equal point 14a on scale 142'' on the opposite end of the skin localizer. One need not mark line 18 on the skin if one does the procedure directly under the laser light sheet from the CT scan as it is thrown across the skin through point 14a.

Now the surgeon has placed the edge 24 of the probe guide 8 so that the bottom end of guide hole 9 is just against the desired entry point 12 in Figure 6. The bottom edge 24 of the clip holder 8 is essentially perpendicular to the scan plane, and this orients the entire stereotactic system relative to the natural coordinate system of the CT machine, namely the scan plane, also referred to as the axial plane, or equivalently, the line perpendicular to the scan plane. In this sense, edge 24 is a reference structure to orient the instrument to the CT plane and insures that the probe will be passed into the body in the CT plane. Other such reference structures, such as orthogonal edges, surfaces, etc., can be constructed, and some others are illustrated below. At this point, when needle 10 is pushed through the entry point 12, then it will lie in the same plane as the image slice seen in Figure 7.

Further appropriate angulation of the approach of needle 10 is achieved by orienting the handle 1 manually so that the bubble level 2 reads at the center. This means that the axis of the handle coincides with the gravitation vertical axis 20. The arc 3 and connected handle 1, can be designed so that leveling of handle 1 to gravity results in the angular graduations 301 on arc 3 also being calibrated to the gravitation vertical 20. Thus when slide 4, which is cooperatively connected to probe guide 8, is set at a given angle on arc 3, then probe 10 will be at that same angle relative to a line parallel to gravitational vertical line 20 when the handle is leveled to gravity. Because the angle slide 4 has been set on the arc graduations at the same angle as 23 in the CT image, then there is an equivalent correspondence between the approach direction of needle 10 relative to the gravitational vertical as the approach direction 22 is to the vertical line 201 in the scan image. At this point, the needle can be pushed into the patient's body, and its path will correspond to line 22 in the CT image. Its tip will hit target 19, when the appropriate depth has been achieved corresponding to the distance between entry point 151 and target point 18. This distance can also be measured on the CT console and can be measured off on the biopsy needle by appropriate depth stops or other markers.

There are several significant features about the design in Figure 6 which are notable in terms of the instruments function. In use, the instrument is designed to rest against the patient's skin very near the entry point. Thin is a significant stabilizing action when trying to line-up the entire system to gravity and the CT scanner as well as to provide movement of the instrument to correspond to the respiratory undulations and other movements of the patient's body. In Figure 6, for example, the lower edge 24 of probe holder 8 is placed against the patient's skin just near the predetermined entry point 12 on the skin. The lower edge 12 then acts as a contact means or fulcrum so that minor variations of the patient's body will cause the fulcrum means 24 to rise and fall or translate side-to side with the patient's body. In that sense, the contact means on the stereotactic instrument to be placed near the entry point removes 3 translational degrees of freedom and greatly simplifies the surgeon's requirements to hold the tip of the needle at the entry point. There is another benefit of this contact means with the patient's body at the entry point. It partially relieves the weight of the instrument by resting on the body, making it easier for the surgeon to concentrate on the leveling procedure using the gravitational leveling means, in this case the bubble level 2. Another significant feature of the design in Figure 6 is the alignment edge 24, which aligns parallel to one of the rods 15 of the localizer and thus guarantees that the probe guide hole 9 will be aligned in the plane of the CT scan. This in turn ensures that the needle 10 will be inserted into the body in the plane of that CT scan. By having this reference edge placed mechanically against the patient's skin as described above, the surgeon does not have to worry about visually and manually monitoring that degree of angular orientation to ensure insertion of the needle into the CT plane. So the combination of the contact means and contact to the patient's body near the entry point and that reference edge means in Figure 6 eliminates three translational degrees of freedom and one angular degree of freedom from the surgeon's conscious efforts in getting the instrument and probe lined-up for its insertion into the body. Now all the surgeon has to be concerned with are the two remaining angular degrees of freedom which are required to align the handle 1 parallel to the gravitational vertical line 20. He can concentrate on keeping the bubble level 2 central in the bubble gauge which assures this alignment of the instrument to gravity. The two angular degrees of freedom associated with this leveling or alignment to gravity correspond to the two orthogonal angles relative to the vertical line 20. Thus, the invention of Figure 6 has the significant advantages of light weight, hand-held, portability with fulcrum means to contact the body and align the instrument to the CT plane in addition to the arc means to achieve the appropriate probe path angle and simple gravitational level means in the form of a single spherical or bubble level to achieve the full alignment of the probe in the direction corresponding to that seen in the CT image. It is notable that using a single bubble level like 2 in Figure 6 is a vast improvement over using two separate linear bubble levels, since the surgeon only has to concentrate on the one bubble rather than two to achieve the orientation relative to gravity. Using a floor-mounted system like that shown in Onik-Cosman-Wells does not achieve the function of the present invention namely to contact and ride with the movement of the patient's body in the vicinity of the entry point.

Figure 8 illustrates how the invention of Figure 6 can overcome the difficulty of the prior art instrument in Figure 4 in achieving stabilization at the entry point for a convoluted condition of the patient's skin. As in Figure 4, the skin 2017 in Figure 8 has a troth in it and the entry point 2012 is at the bottom of the troth. The bottom edge of the clip-holder 2024 coincides with the entry point 2012 and thus one can achieve contact and a pivot action around the entry point 2012 of the entire instrument. One notices that the graduated arc 2003 is completely clear of the body. As explained above, this is not possible with the prior art. It is possible with the present instrument partially because the probe guide 8 and its lower edge 24 in Figure 6 are extended out in the direction that the probe will be passed so it is the most proximal portion of the instrument at the point of entry and contact with the skin.

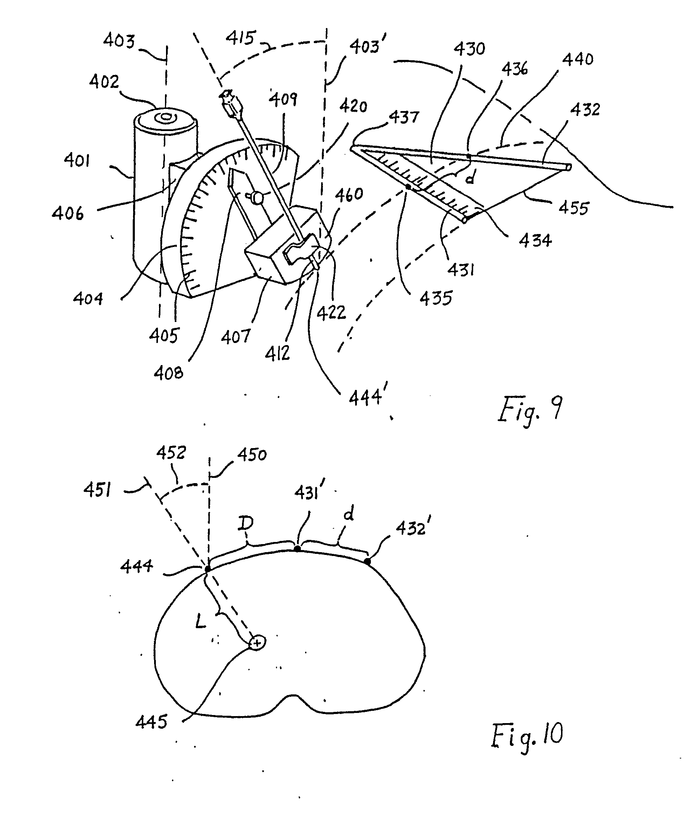

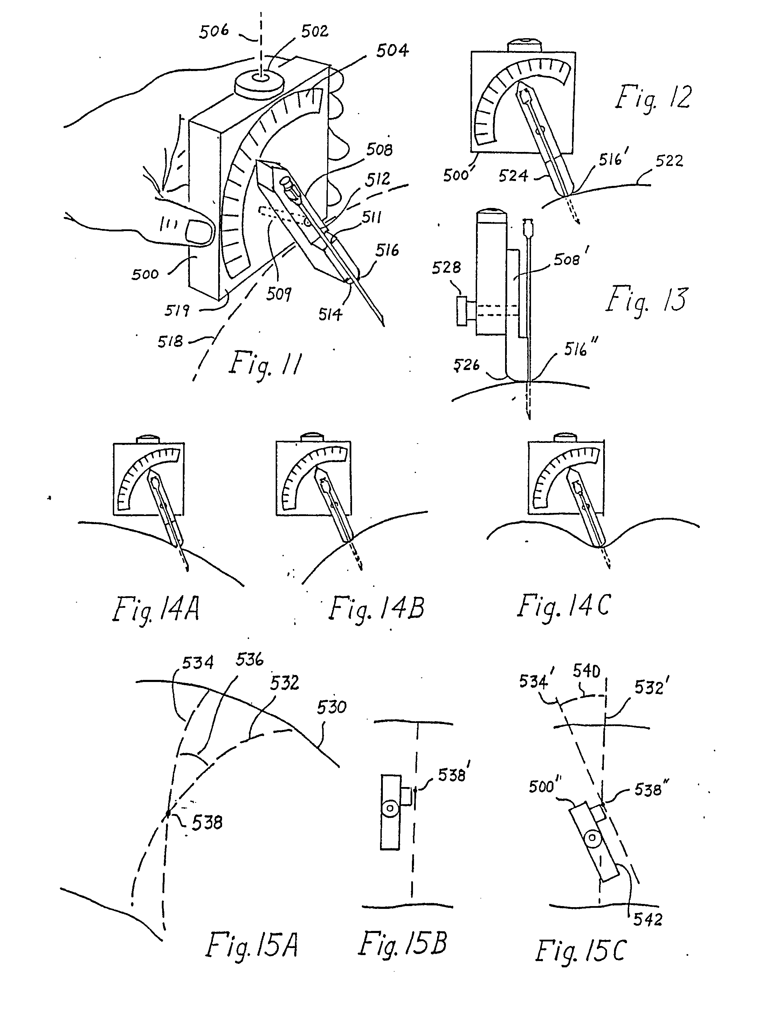

Figure 9 and Figure 10 illustrate another embodiment of the invention and method as well as another method for determining an entry point from the CT scan onto the patient's body. Figure 6 illustrated the use of a multiple rod localizer ladder that had a triangle on one end. This is not the only way of determining an entry point on the patient's body from a localizer. For instance, in the patent by Onik, Cosman, and Wells, it is discussed how one can use a single triangular localizer to determine the position, distance, and coordinates of an entry point on the patient's skin which is at a distance removed from the localizer. In Figure 9 a further embodiment is shown of the present invention, and a method is illustrated wherein the entry point is at a distance from the localizer and is determined from simple distance measurements from the CT scan. In Figure 9 the localizer again has a handle portion 401 and a bubble-level on the top 402 which establishes the alignment of the handle axis to the gravitational vertical line 403. The vertical arc 404 is as in the pervious examples, with angular graduation markings on it 405. The vertical arc is attached to handle by a fixed means 406. The probe carrier block 407 can pivot on a bearing relative to arc 405, and the angular slide 408 indicates the angle at which the probe block and the probe 409 is set relative to the gravitational vertical. In other words, probe line 409 which is guided in the probe guide groove 412 is set relative to the gravitational vertical line $403'$ at an angle 415. The probe slide can be fixed in a given position by knob 420. Also, when a needle shaft or probe such as 409 is set into the probe slide groove 412, then it can be captured by a retainer such as 422, which may be spring-loaded or quick-release in nature.

The skin localizer in Figure 9 is represented by the triangle 430 which again has two sides 431 and 432 which show up on the CT scan. It may be set on the skin with its base edge 455 parallel to the CT slice plane using the CT laser aligning lights. In Figure 10 the images of the radiopaque edges or rods of the triangle corresponding to 431 and 432 are represented by $431'$ and $432'$ as dot images. The distance between these images is measured on the CT console from the CT image in Figure 10 represented by d between the points $431'$ and $432'$. By determining this distance d, the "level" or slice position of the CT scan is also determined. This is equivalent to determining the points 435 and 436 where the CT scans intersects the triangle legs 431 and 432, respectively. For instance, if the triangle is a 45-degree right triangle, then the distance d between 435 and 436 is equal to the distance between the apex 437 and point 435. The scale 434 can read out this 437 to 435 distance, and thus immediately indicates the scan point by knowing d. The dashed line 440 indicates where the CT scan would cross the skin of the patient. Once the intersection point 435 is known the dashed line 440 can be drawn in pen on the patient's skin by using the laser lights of the CT scanner by passing the CT laser lights through the point 435. The CT laser lights then project on the skin the rest of the points where the CT scan hits the skin which in turn can may or may not be marked with a pen.

To illustrate how this skin localizer, in contrast to the ladder-type localizer of Figure 6, can be used to determine an entry point, a point 444 on the CT image of Figure 10, is marked as the desired entry point, to approach the target 445 the in depth of the body. From the CT console the distance between the points 444 and 431' is then measured using the standard CT console software. This distance is designated by D in Figure 10. That distance D can then be simply measured off on the patient's body from the point 435 along the line 440 (which has been marked on the skin) by means of a protractor or other linear measuring device to determine the desired physical entry point 444' on the patient's skin as shown in Figure 9. Also illustrated in Figure 10, the standard CT scan console software enables a vertical line 450 to be drawn on the CT image. The line 451 is also shown between our chosen entry point 444 and the target 445 as shown in Figure 10. Line 451 subtends an angle 452 relative to the vertical line 450. An equal angle 452 can then be set on the vertical arc 404 of the stereotactic instrument by means of arc slide 408. This determines that angle 415 between the probe line and the gravitational vertical line 403' (which is parallel to 403) is equal to the angle 452.

The stereotactic instrument can now be brought into contact with the patient's body. The bottom edge of probe guide 407 contacts the patient's body and acts as a fulcrum for leveling the stereotactic instrument. The tip of the probe 409, or equivalently the end of the probe guide channel 412 is moved exactly over the predetermined entry point 444'. The reference surface (or reference edge) 460 is aligned parallel to the line 440 which has been marked on the patient's skin or is cast on the skin by laser lights, and represents the intersection of the scan plane with the patient's skin. This assures that the probe 409 will pass into the plane of the scan image seen in Figure 10 when the probe is inserted into the body. We are assuming that the CT scan plane is with an untilted gantry, that is, it is vertical slice relative to gravity. Having used the lower surface 407 as a fulcrum or contact against the patient's skin with the probe guide hole over the entry point, and the reference surface 460 aligned to the scan plane line 440, the surgeon can now orient the handle 401 such that the bubble level 402 is centered. This aligns the last two degrees of freedom, the two angular degrees of freedom around line 403, which then assures that the direction of probe 409 corresponds to the probe path 451 in Figure 10. At this point, the setting of the slide 408 on the angle dial 405 represents the angular deviation 405 of the probe line from the gravitational vertical line 403'. The probe can then be pushed through the entry point in the skin, and when it has traversed a depth corresponding to the distance L indicated in Figure 10, the tip of the probe will be at the physical target corresponding to the target image 445 in Figure 10. Again, the depth L can be measured from the CT image using the standard CT scanner console software, i.e., using the two point distance function which is common to all CT scanner consoles.

There are many variations of the presently described invention that could be thought of by those skilled in the art. Simplified arc systems such as a sextants or protractors could be devised with an in-dwelling bubble level system. The gravity leveling system does not have to be a bubble-level. It could be a pendant or plum-bop type of mechanical device which hangs in a column or is registered to a reference marker. The single bubble-level is particularly practical since it simply aligns two angular movements with one visual setting. The bubble-level in Figure 6 could actually be removed from the shaft 1 so that the entire device could be steam autoclaved and then the bubble level put back in when needed. The devices could be made of plastic or metal. Plastic devices would lend themselves to being disposable for one-time use. Metal devices would lend themselves to resterilization. Many kinds of probe guides can be devised such as clip holders, guide tubes, guide channels, etc. Other configurations of the angle dials and angle slides can be thought of. Many different means for providing references edges of reference sighting means to align the instrument to the natural CT machine axis or CT plane can also be devised. The instrument can be made MRI compatible so it can be used with MRI tomographic scanners as well as CT scanners.

The instrument could be referenced to the CT image and to the body by a alignment rod scheme connected to the body or by laser lights on the CT scanner or aligned to the scanner. In this case, the bubble level scheme of the Figure 6 and 9 could be replaced by alignment rods or alignment reference edges aligned relative to the patient fixed or machine fixed reference lines. For instance, in Figure 6 rather than having the bubble level 2, the handle 1 could be aligned to crossing laser lights that shine over the patient and are fixed to the CT machine. In this way, the establishment of a reference axis for the handle 1 could be determined without a gravity referenced vertical bubble level. The means for reference orientation of the hand-held stereotactic device could be done in many ways. Although bubble levels have been shown in the examples above to determine the gravitational vertical line system, other schemes whereby the instrument can be aligned to the gravity or to axis established relative to the patient or the CT scanner are included in the scope of this invention.

The entry point of the probe on the skin can be determined in many ways not specifically shown here. For instance, discrete radiopaque markers or linear elements can be placed on the patient's skin where the entry point has been calculated to be. The skin localizer ladder or triangle described above are convenient, but could be replaced by other means. Thus, this application claims the use of the hand-held stereotactic instrument no matter what means are used to determine the entry point, either with a skin localizer or by having predetermined the desired entry point in some other way. For instance, the entry point could be located by using the laser lights of the CT scanner or other ancillary laser light devices, which would not require any mechanical skin localizer at all.

The elements of the patient contact means or fulcrum means and the reference or alignment means to the CT scanner plane can be implemented in a variety of ways. The patient contact means or fulcrum means can be pad or edge structures in the proximity of the probe guide end which will contact the patient's skin near the predetermined entry point. These can take various shapes to conform to the patient's body and may have sticky or rough surfaces to prevent lateral slippage of the contact point relative to the skin. They may have specific attachment means to lock to a skin localizer which may have been stuck to the patient's skin to prevent any translational movement once the leveling procedure begins. The reference edge means of alignment means described in the examples above may take the form of rods, edges, cross hairs, etc. which can be lined up by sight with laser lights associated with the CT scan plane or lines and markings on the patient's skin either along the intersection of the CT scan plane with the skin or orthogonal to that direction.

It has been noted above in the examples that it is advantageous to have to contact means or fulcrum means on the stereotactic instrument very near, if not at, the point where the probe guide contacts the entry point. In this way, the pivoting action about that fulcrum or contact point is around the entry point itself. This is important since the direction of the surgical probe or needle which is guided by the probe guide will then pivot about the entry point as the stable fixed point to which it must pass. Any differential movement of the skin, as in respiratory undulations, will therefore be irrelevant since the probe path still passes through the predetermined desired entry point. Thus, a favorable embodiment of the invention comprises contact means which is integrated with the probe guide means to contact the skin near the entry point. The contact means may be a distance from the desired entry point and still enable the same fulcrum or stabilizing effect. Thus we also claim here use of contact means that are not at the entry point. Analogously, the reference means for aligning the instrument to the CT plane is best placed in proximity to, if not integrated with, the probe guide and fulcrum means. These elements, in fact, could be integrated into one structure so that a single structure serves the three functions of guiding the probe, providing the fulcrum, and providing the alignment with the scan plane. Thus, although these important functions are talked about separately in this application, they may be unitized into one structure. The principle illustrated by this invention of stabilizing the hand-held instrument on the patient's skin at the entry point, while also stabilizing the orientation by contact and reference means against the patient's body so as to remove the three translational degrees of freedom associated with the contact and the one angular degree of freedom associated with the CT plane alignment, greatly simplifies the ability of the surgeon to make the final gravitational reference alignment by the bubble-level. This means, that our instrument reference point moves with the patient's skin at the entry point. The surgeon now only has to concentrate on the bubble-level or other gravitational alignment means to make his final orientation of the entire instrument. It is also added parenthetically that by having the probe holder in direct contact with the entry point, simplified depth measurements for passing the probe to the target can be implemented. For example in Figure 6, if the width in the probe direction of probe guide holder 8 corresponds to an integral number of centimeters, then a depth stop can easily be placed on the shaft of needle 10 to correspond to a given probe penetration into the body to correspond to the distance between points 151 and target 19 in Figure 2.

It is not possible for the embodiments of the previous prior art inventions shown in Figures 1 and 3 to have their contact or fulcrum point near the entry point of the skin. In Figure 2, for example, it is not satisfaotory to argue that the contact of the needle point 1077 at the entry point could act as a satisfactory fulcrum in all cases. This is because needles may be a very fine gauge and thus very flexible so that they are not adequate to rigidly stabilize the instrument at the entry point for the purpose of acting as a fulcrum or pivot point.

Figure 11 shows a particularly simple embodiment of the present invention. It consists of a body 500 in the form of a rectangular block with the bubble-level 502 embedded in the top of it so that when the circular level is balanced to gravity then the block stands straight up vertically. Engraved on the side of the block is angle dial 504, which when the block is aligned to gravity by the circular level 502, the dial corresponds to a calibrated angular departure from a gravitational vertical line parallel to line 506. The angle slide 508 pivots and can be locked in position by pivot bearing 509. The probe guide groove 511 enables the probe 512 to slide in the groove towards the direction of the target. The rounded bottom surface 514 can contact the patient's skin at the entry point 516. A dashed line 518 indicates the intersection of the CT slice with the patient's skin. This may be drawn on the patient's skin with an ink pen using the CT laser lights or maybe the image of the laser lights directly on the skin if the instrument is held near the laser lights. Aligning the face 519 of the block 500 to this line assures that the probe guide 511 will direct the probe 512 in the plane of the scan if the scan is an axial scan or non-tilted scan.

Figure 12 shows the instrument of Figure 11 in a vertical view as viewed in a CT slice plane. Point 516$'$ is the entry point on the patient's skin 522 and because the probe guide protrusion 524 extends well out beyond the margin of the block 500$'$ then this contact can be made no matter what the curvature or undulation of the patient's skin.

Figure 13 shows a side view looking parallel to the direction of the CT plane and one sees that the bottom portion 526 of the contact point can be rounded so as to made it less uncomfortable for the patient and further get into more point-like contact at the entry point 516$''$. Notice that thumbnut 528 may be used to lock the angle dial 508$'$ at its proper angular position.

Figure 14a, 14b, and 14c illustrate how the instrument of Figure 11 can contact the patient's skin at extreme slope angles of the skin which would be impossible with inventions of the prior art such as in Figure 1. These correspond to the analogous examples which were illustrated for the prior art in Figures 2, 4, and 5.

Figure 15a, 15b, and 15c illustrate a means of using the simple instrument of Figure 11 to achieve compound angular approaches which would be derived from "out of plane" target and entry point probe paths. Such out of plane paths were describe extensively in patents by Onik, Cosman, and Wells referenced above. Consider, for example, the diagram in Figure 15a which shows the patient's body 530 and an entry point 538 which is visualized in a CT slice. The projection of that CT slice on the patient's skin is indicated by the dashed line 532. Now, as explained in Onik, Cosman, and Wells, if there is a second slice in which one sees the desired at depth in the patient's body, then to pass a probe through the entry point 538 to that target seen in the second slice one would need to direct the prcbe in a trajectory which is out of the first scan slice that gave rise to the intersection line 532. This can be referred as an "out of plane" trajectory, meaning that the needle tip extends out of the plane which contains the entry point. It is thus a compound angular trajectory in the sense that one cannot use a single angle in the CT scan slice of the entry point such as set on the instrument in Figure 11, for instance. Indeed, to pass a probe through point 538 to a targeted depth using an instrument like that in Figure 11, one would have to determine two angles; the first being an angle set on the instrument in Figure 11, and the second and angle such as angel 536 in Figure 15a. This angle 536 is between the axial intersection line 532 and the second line indicated by dashed line 534 which is at an azimuthal angle of a plane rotated from the axial plane about a vertical axis, and rotated by an azimuthal angle which can be determined mathematically by knowing the coordinate differences between the entry point and the target point. This is explained in the patent by Onik, Cosman, and Wells. That line 534 may be determined in a variety of ways by projections of a protractor lined up on line 532 and projected down vertically onto the patient's body at the entry point 538. Once this projected line 534 is known, one can use the simple instrument of Figure 11 once again to achieve this out of plane or compound angle approach. Figure 15b shows again looking down on the patient how the instrument of Figure 11 would be used for an axial slice such as described above or corresponding to the entry point being in the same vertical CT plane as the target point. Figure 15c indicates how the instrument, again looking down vertically from above on the patient, can be used at an angle of 540 relative to the line 532$'$ and 534$'$. That angle 540 as one used directly from above can be referred to as the azimuthal angle and can be projected and described onto the patient's body by a simple protractor or other projection means. Now one lines up parallel the edge 542 of the body 500$'$ of the stereotactic instrument to the line 534$'$. This achieves the compound angle approach. Now the angular setting of the vertical arc on the instrument achieves the second angular degree of freedom. This sends the probe out through the entry point 538″ to a target at depth but out of the entry point plane indicated by the dashed line 532′. Thus, the instrument of Figure 11 can be used for more sophisticated approaches between a predetermined entry point and a predetermined target point in depth. The plane that it sends the probe into does not have to be parallel to either the plane of the entry point nor the plane of the target point. The simple solid geometry to determine the compound angles mentioned above is done in an elementary fashion once the target and entry point have been identified in predetermined slices of the CT scanner.

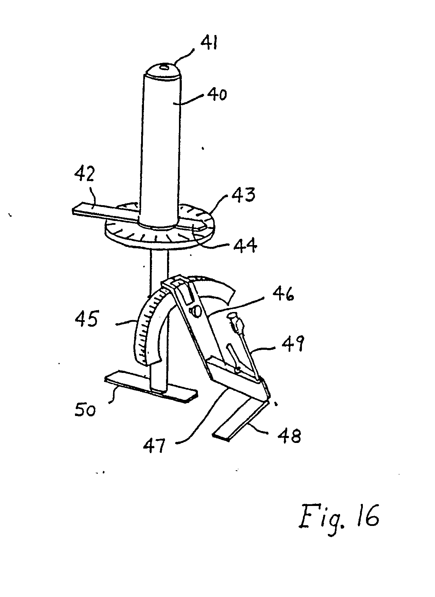

Figure 16 shows yet another embodiment of the present invention which has built-in the two angular degrees of freedom needed for out-of-plane approaches. Here, the vertical arc 45 plus clip holder 47 and arc slide 46 correspond to the analogous ones in Figure 6 and 11. An addition there is the arc 43 and rotational elements 42 and 44 which enable the orientation of the probe guide or clip holder 47 and the vertical arc 45 to be further angulated relative to the natural scanner axes. For instance, in this situation, the natural scanner axis might be aligned parallel to elements 42 and 44. By setting a given horizontal angle of dial point of 44 relative to angle dial 43, compound angular relationships of the probe path 49 relative to the natural scanner axis (that is, the plane parallel to the axial plane or an axis perpendicular to the CT axial plane) can be achieved. As described in the Patent of Onik, Cosman, and Wells, this can give rise to compound angulations, which means that an entry point can be selected in a different CT slice from the target point, and still, the mathematical path which passes through both can be determined. The bubble level 41 and handle 40 are analogous to those in Figure 6 and establish the gravitational vertical relationship. Thus, it is possible as illustrated in Figure 16 to have a hand-held, gravitationally aligned device which can give compound angles by means of two angular degrees of freedom corresponding to the arcs 43 and 45. The pad 48, which is at the lower margin of the clip holder 47, has an edge which is perpendicular to the lower edge of clip holder 47. Thus that edge could be lined up parallel to the intersection of the scan plane with the body as illustrated by the dashed line in 18. This is yet another way of establishing a reference orientation of the device relative to the patient's body and to the skin localizer as illustrated in Figure 6. It also illustrates a larger area contact means to act as a fulcrum against the body for stability.

## Claims

1. A hand-held stereotactic instrument for directing a surgical probe into the living body comprising:
   a) angle defining means with:
      i) graduation means;
      ii) index means so that said angle graduation means and said index means can be oriented relative to each other so that said index means can be lined up at a predetermined angle on said angle graduation means;
   b) leveling means cooperatively coupled to said angle defining means so that said angle defining means can be leveled relative to gravity;
   c) probe guide means that can be cooperatively coupled to said angle defining means and which defines a probe axis and which is adapted to guide said surgical probe in substantially the direction of said probe axis, so that when said angle defining means is leveled to gravity and said index means is set at said predetermined angle relative to said angle graduation means, then said probe axis and accordingly said surgical probe will be directed at said predetermined angle relative to said gravitational reference line;
   d) body contact means cooperatively coupled to said probe guide means and so adapted that when said angle defining means is leveled relative to gravity, then, as seen in at least one side elevation view of said stereotactic instrument, said body contact means protrudes substantially from the rest of said stereotactic instrument in substantially the same direction as said probe axis for any value of said predetermined angle set on said angle defining means;
   whereby, when in use, said stereotactic instrument is so adapted that it can be held by hand and brought near to said living body so that said body contact means will contact said living body near a predetermined entry point on said living body, and said surgical probe can be guided by said probe guide means in the direction of said probe axis to aim at said predetermined entry point, and said stereotactic instrument can be oriented by hand while said body contact means acts to stabilize as with a fulcrum said stereotactic instrument against said living body near said predetermined entry point so that said angle defining means can be leveled relative to gravity by said leveling means, and whereby said surgical probe can be introduced into said living body through said predetermined entry point at said predetermined angle relative to said gravitational reference line.

2. The apparatus of Claim 1 wherein said leveling means comprises a bubble level.

3. The apparatus of Claim 1 adapted for directing said surgical probe based on images from a tomographic scan, and further comprising:

reference structure means having a linear edge structure and cooperatively coupled to said stereotactic instrument and so adapted that, when the plane of said tomographic scan is known relative to said living body and marked by a reference line on the skin of said living body that said reference structure means can be used to align said stereotactic instrument relative to said plane by aligning said linear edge structure to said marked reference line, whereby when said surgical probe is introduced into said living body, it will lie in said tomographic plane.

4. A hand-held stereotactic apparatus for directing surgical probes into the living body based on an image from a tomographic scan through said living body comprising:

a) reference means to be aligned relative to the gravitational reference line;

b) leveling means cooperatively coupled to said reference means to enable alignment of said reference means to said gravitational reference line;

c) angular arc means with angular graduations on it for establishing an angular reference relative to said reference means and;

d) probe guidance means which defines a probe axis and which is adapted to guide said surgical probe in substantially the probe axis direction, and which can be set relative to said angular arc means so that said probe axis can be oriented with a predetermined angle to said reference means;

e) body contact means adapted to contact said living body when said surgical instrument is in use and to act as a fulcrum against said living body to facilitate aligning said reference means to said gravitational reference line, said body contact means being so adapted that when said reference means is leveled to gravity then, as seen in at least one side elevation view, said body contact means is seen to project from the rest of said stereotactic instrument with a direction of projection substantially coincident with the probe axis as seen in said elevation view for any set value of said predetermined angle;

f) whereby said reference means, leveling means, arc means, probe guide means and body contact means are so cooperatively connected that when said stereotactic apparatus is held by hand in proximity to the living body that said reference means can be aligned to said gravitational reference line while said stereotactic instrument is being stabilized by said body contact means in contact with said living body, and while said surgical probe, placed into said probe guidance means, can be oriented relative to the said gravitational reference line by said predetermined angle which has been set by said probe guide means relative to said angular arc means and aimed at a predetermined entry point through which it is desired that said surgical probe to pass, and whereby said surgical probe can be subsequently introduced into said living body through said entry point at said predetermined angle relative to said gravitational reference line, said predetermined angle having been determined from said tomographic scan images to be the correct angle of said probe relative to said gravitational reference line to reach a target in said living body.

5. The apparatus of Claim 1 wherein said leveling means comprises a bubble level system for orienting said reference means to the gravitational vertical line.

6. The apparatus of Claim 4 and further comprising a reference edge means so that when said stereotactic instrument and said surgical probe means are set against said living body and when a predetermined plane in which said surgical probe should be passed into said living body is known relative to said living body by a reference line on the surface of said living body, that said reference edge means makes it possible to introduce said surgical probe into said living body within said predetermined plane by alignment of the reference edge structure to said reference line of said predetermined plane.

7. A hand-held stereotactic instrument for inserting a surgical probe into the living body to hit a target in the living body comprising:

a) a vertical arc means with angular graduations on it;

b) leveling means cooperatively connected to said vertical arc means to align said vertical arc means related to a gravitational reference line;

c) probe guide means which is adapted to guide a surgical probe that will be directed into said living body, the probe guide means being so adapted to be attachable to said vertical arc means and to be aligned relative to said vertical arc means according to said angular graduations; and a portion of said probe guide means being adapted to contact said living body and thereby act as a pivot point of said stereotactic instrument against said living body;

said vertical arc means, leveling means and probe guide means being so cooperatively connected that in use when said leveling means is used to align said vertical arc relative to said gravitational vertical line, then when said probe guide means is set at a predetermined angle as measured by said graduation means on said vertical arc that said probe guide means will guide a probe at said predetermined angle relative to said gravitational reference line; and whereby, when in use, said hand-held stereotactic instrument can be held in the hand of the surgeon so that said portion of said probe guide means is made to contact said living body so as to stabilize said stereotactic instrument against said living body while manually manipulating said leveling means to align said vertical arc means to said gravitational reference, and said probe can be directed by said probe guide means into the said living body at an angle relative to said gravitational reference line corresponding to said predetermined angle.

8. The apparatus of Claim 7 adapted to direct said probe into said body based on tomographic image data from a tomographic slice through said body, and further including reference means so connected and so adapted to said stereotactic instrument that when in use, the plane of said tomographic slice is determined relative to said living body, said reference means can be used to orient said stereotactic instrument so that said probe guide means can direct said probe into said living body in said plane of said tomographic slice, and when in use said predetermined angle can be determined from said tomographic slice data.

9. A method for directing a probe into a living body to reach a physical target point within the body by means of a tomographic image from a tomographic scan through said living body comprising the steps of:

a) placing a localizer in a fixed position relative to said living body where at least a portion of said localizer being adjacent to the skin of said living body such that a tomographic scan through said localizer will give an image entry point location on the scan image which determines a physical entry point on the patient's skin adjacent to said localizer;

b) taking a tomographic image through said body and through said localizer to produce a tomographic image from which said physical entry point on the skin can be determined;

c) identifying a target image point in said tomographic image which corresponds to said physical target point and from which the spatial relationship of said target image point relative to said image entry point can be determined;

d) calculating a trajectory in space relative to the plane of said tomographic scan which passes through said physical entry point on the skin and through said physical target point and calculating the distance along said trajectory between said physical entry point and said physical target point;

e) and using a hand-held stereotactic instrument to guide said probe along said calculated trajectory, such that said probe will pass through the skin at said physical entry point to reach said physical target point.

10. A method of stereotactically placing a probe into a living body to reach a target seen on a tomographic image, comprising the steps of:

a) taking a tomographic image of said living body and determining from said tomographic image a target point in said living body and an entry point near the surface of said living body through which said probe is to pass to said target point;

b) determining an image reference line in said tomographic image corresponding to said gravitational reference line relative to said living body;

c) determining a path angle between said image reference line and the image path line which corresponds to the angle between said gravitational reference line and a physical path line between said entry point and said target point;

d) using a hand-held stereotactic instrument comprising;

i) angle defining means with angular graduations;

ii) probe guide means cooperatively connected to said angle defining means which when set at said path angle relative to said angular graduations will guide said probe along said physical path line at said path angle relative to said gravitational reference line when said angle defining means is leveled relative to gravity;

iii) leveling means cooperatively connected to said angle defining means for leveling said arc means relative to gravity; and

e) manually bringing said hand-held stereotactic instrument in proximity to said living body such that said probe guide means contact said living body, leveling said angle defining means relative to gravity while using said probe guide means in contact with said living body as a stabilizer against movement of said stereotactic instrument relative to said living body, and passing said probe in said probe guide means into said living body along said physical path line through said entry point in the plane of said tomographic image to hit said target seen in said tomographic image.

11. A method of stereotactically placing a probe into the living body to reach a target seen on a tomographic image, comprising the steps of:

a) taking a tomographic image of said living body and determining from said tomographic image a target point in said living body and an entry point near the surface of the living body through which said probe is to pass to said target point;

b) determining an image reference line in said tomographic image which corresponds to a physical reference line relative to said living body;

c) determining a path angle between said image reference line and the image path line which corresponds to the angle between said gravitational reference line and a physical path line between said entry point and said target point;

d) using a hand-held stereotactic instrument comprising:

i) angle defining means with angular graduations;

ii) probe guide means cooperatively connected to said angle defining means which when set at said path angle relative to said angular graduations will guide said probe along said physical path line at said path angle relative to said gravitational reference line when said angle defining means is leveled relative to gravity;

iii) leveling means cooperatively connected to said angle defining means for leveling said angle defining means relative to gravity, and

iv) reference means cooperatively coupled to said probe guide means for allowing alignment of said probe in said probe guide so that probe will pass within the plane of said tomographic image; and

e) manually bringing said hand-held stereotactic instrument in proximity to said living body such that said probe guide means contacts said living body, leveling said angle defining means relative to gravity and aligning said probe by means of said reference means to be within said plane of said tomographic image, while using said probe guide means in contact with said living body as a stabilizer against movement of said stereotactic instrument relative to said living body, and passing said probe in said probe guide means into said living body along said physical path line through said entry point in the plane of said tomographic image to hit said target seen in said tomographic image.

12. The method of Claim 9 and further comprising the step of placing a localizer on the skin of said human body so that said tomographic image will enable the said entry point to be determined relative to said localizer, and whereby said stereotactic instrument can be positioned relative to said localizer so that said probe guide means can direct said probe through said entry point to pass to said target along said physical path line.

13. The method of claim 11 and further comprising the step of placing a localizer on the skin of said human body so that said tomographic image will enable said entry point to be determined relative to said localizer, and whereby said stereotactic instrument can be positioned relative to said localizer so that said probe guide means can direct said probe through said entry point to pass to said target along said physical path line.

14. A method for directing a probe into a living body to reach a physical target point within the body by means of a tomographic image from a tomographic scan through said living body comprising the steps of:

a) placing a localizer in a fixed position relative to said living body where at least a portion of said localizer being adjacent to the skin of said living body such that a tomographic scan through said localizer will give a first image point on the scan image which determines the first physical point on the patient's skin adjacent to said localizer;

b) taking a tomographic image through said body and through said localizer to produce a tomographic image from which said first physical point on the skin can be determined and from which a second image point on the skin can be determined on said tomographic image;

c) measuring the distance between said first image point and said second image point to determine an associated second physical point on the skin corresponding to said second image point in said tomographic image;

d) identifying an image target point in said tomographic image corresponding to said physical target point and from which the spatial relationship of said image target point relative to said image entry point can be determined;

e) calculating a probe trajectory in space relative to the plane of said tomographic scan which passes through said physical entry point on the skin and through said physical target point and calculating the distance along said trajectory between said physical entry point and said physical target point;

f) and using a hand-held stereotactic instrument comprising a vertical angle defining means with a graduated angle scale on it, a probe guide means cooperatively coupled to said angle defining means which can be set at a predetermined angle relative to a gravitational reference line by said graduated angle scale when said stereotactic instrument is leveled relative to gravity, leveling means to level said stereotactic instrument to gravity, said probe guide means adapted to direct said surgical probe into said body at said predetermined angle, reference means cooperatively connected to said probe guide means which can enable said probe to be inserted within the plane of said tomographic scan, the method involving bringing said hand-held stereotactic instrument by hand into proximity to said living body so that probe guide means will approximate said second physical point, and setting said probe guide means on said vertical arc to correspond to said calculated probe trajectory and leveling said stereotactic instrument by means of said leveling means to align it to said gravitational reference line, aligning said reference means relative to the human body, so that said probe will pass within said plane of tomographic scan; and inserting said surgical probe along said probe guide into said body through said second physical point to a depth corresponding to said distance between said second physical point and said physical target point, whereby the tip of said surgical probe will pass through said physical target point.

15. A method for directing a surgical probe into the living body to reach a physical target point within the body by means of a tomographic image from a tomographic scan through said living body comprising the steps of:

a) placing a localizer in a fixed position relative to said living body where at least a portion of said localizer being adjacent to the skin of said living body such that a tomographic scan through said localizer will give a first image point location on the scan image which determines the first physical point on the patient's skin adjacent to said localizer;

b) taking a tomographic image through said body and through said localizer to produce a tomographic image from which said first physical point on the skin can be determined and from which a second image point on the skin can be determined on said tomographic image;

c) measuring the distance between said first image point and said second image point, to determine an associated second physical point on the skin corresponding to said second image point on the tomographic image;

d) identifying a target image point on the image in said tomographic image corresponding to said physical target point and from which the spatial relationship of said target image point relative to said second image point can be determined;

e) calculating a trajectory in space relative to the plane of said tomographic scan which passes through said second physical point on the skin and through said physical target point and calculating the distance along said trajectory between said second physical point and said physical target point;

f) using a hand-held stereotactic instrument to guide said surgical probe along said calculated trajectory, such that said probe will pass through the skin at said second physical point and reach said physical target point.

16. A method for directing a surgical probe into the living body to reach a physical target point within the body by means of a tomographic image from a tomographic scan through said living body comprising the steps of:

a) placing a localizer in a fixed position relative to said living body where at least a portion of said localizer being adjacent to the skin of said living body such that a tomographic scan through said localizer will give a first image point location on the scan image which determines the first physical point on the patient's skin adjacent to said localizer;

b) taking a tomographic image through said body and through said localizer to produce a tomographic image from which said first physical point on the skin can be determined and from which a second image point on the skin can be determined on said tomographic image;

c) measuring the distance between said first image point and said second image point, to determine an associated second physical point on the skin corresponding to said second image point on said tomographic image;

d) identifying a target image point on the image in said tomographic image corresponding to said physical target point and from which the spatial relationship of said target image point relative to said second image point can be determined;

e) calculating a trajectory in space relative to the plane of said tomographic scan which passes through said second physical point on the skin and through said physical target point and calculating the distance along said trajectory between said second physical point and said physical target point.

17. A hand-held stereotactic instrument for directing a surgical probe into the living body comprising;

a) angle defining means comprising;

i) angular graduation means which is so constructed that it defines an angle axis about which angles of said angular graduation means are defined;

ii) index means so adapted that said angular graduation means and said index means can be oriented relative to each other so that said index means can be lined up at a predetermined angle on said angular graduation means;

b) leveling means cooperatively coupled to said angle defining means so that said angle defining means can be leveled to gravity, and when said angle defining means is so leveled, said angle axis is perpendicular to the gravitational vertical line;

c) probe guide means that is cooperatively coupled to said angle defining means and which defines a probe axis which is adapted to guide said surgical probe in substantially the direction of said probe axis, so that when said angle defining means is leveled to gravity and said index means is set at said predetermined angle relative to said angular graduation means, then said probe axis and accordingly said surgical probe will be directed at said predetermined angle relative to the gravitational vertical line;

d) body contact means cooperatively coupled to said probe guide means and so adapted to change its orientation for changes in said predetermined angle so that, when said angle defining means is leveled to gravity and said stereotactic instrument is viewed from a side elevation view from the direction of said angle axis, said body contact means is seen as elongated in shape and protruding substantially in the direction of said probe axis for all settings in the range of said predetermined angles, so that said body contact means is the first part of said stereotactic instrument to contact a plane which is perpendicular to said probe axis and which approaches said stereotactic instrument from said probe axis direction,

whereby, when in use, said stereotactic instrument is so adapted that it can be held by hand and brought near to said living body so that said body contact means will contact the body near a predetermined entry point on said living body, and said surgical probe can be guided by said probe guide means in the direction of said probe axis to aim at said predetermined entry point, and said stereotactic instrument can be oriented by hand while said body contact means acts to stabilize as with a fulcrum said stereotactic instrument against said living body near said predetermined entry point so that said angle defining means can be leveled relative to gravity by said leveling means, and whereby said surgical probe can be introduced into said living body through said predetermined entry point at said predetermined angle relative to said gravitational reference line.

18. The apparatus of Claim 17 wherein said leveling means comprises a bubble level.

19. The apparatus of Claim 17 adapted for directing said surgical probe based on images from a tomographic scan, and further comprising:

reference structure means having a linear edge structure and cooperatively coupled to said stereotactic instrument and so adapted that, when the plane of said tomographic scan is known relative to said living body and marked by a reference line on the skin of said living body that said reference structure means can be used to align said stereotactic instrument relative to said plane by aligning said linear edge structure to said marked reference line, whereby when said surgical probe is introduced into said living body, it will lie in said tomographic plane.

20. The apparatus of Claim 7 in which, when said stereotactic instrument is leveled to gravity, said angle defining means defines a vertical plane perpendicular to said angle axis, and said probe guide means and said body contact means can be coupled to said angle defining means on either side of said vertical plane, so that said probe axis can accordingly be positioned on either side of said vertical plane; whereby direction of said surgical probe into said living body can be easier for right or left handed surgeons or in cases where considerable surface convolutions of said living body exists at said predetermined entry point.

*Fig. 1* (PRIOR ART)

*Fig. 2* (PRIOR ART)

*Fig. 3* PRIOR ART

*Fig. 4* (PRIOR ART)

*Fig. 5* (PRIOR ART)

Fig. 6

Fig. 7

Fig. 8

Fig. 9

Fig. 10

Fig. 11

Fig. 12

Fig. 13

Fig. 14A

Fig. 14B

Fig. 14C

Fig. 15A

Fig. 15B

Fig. 15C

Fig. 16

European Patent
Office

**PARTIAL EUROPEAN SEARCH REPORT**
which under Rule 45 of the European Patent Convention
shall be considered, for the purposes of subsequent
proceedings, as the European search report

Application number

EP 90 11 5695

## DOCUMENTS CONSIDERED TO BE RELEVANT

| Category | Citation of document with indication. where appropriate, of relevant passages | Relevant to claim | CLASSIFICATION OF THE APPLICATION (Int. Cl 4) |
|---|---|---|---|
| D,X | US-A-4 733 661 (PALESTRANT) | | A 61 B 19/00 |
| | * Figures 12,7; column 8, lines 8-15 * | 17,18, 19 | |
| A | | 1-8,20 | |
| | -- | | |
| A | US-A-3 073 310 (MOLARSKI) | | |
| | * Figure 8; column 2, lines 40-44 * | 1,3,4, 6-8,19 | |
| | -- | | |
| D,A | US-A-4 838 265 (COSMAN) | | |
| | * Figure 1; column 3, lines 18-24 * | 3,4,8, 19 | |
| | -- | | |
| | ./. | | |

**TECHNICAL FIELDS SEARCHED (Int. Cl.4)**

A 61 B

## INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely:     1-8,17-20

Claims searched incompletely:

Claims not searched:     9-16

Reason for the limitation of the search:

Method for treatment of the human
or animal body by surgery or therapy
(See art. 52(4) of the  European
Patent Convention)

| Place of search | Date of completion of the search | Examiner |
|---|---|---|
| THE HAGUE | 14-11-1990 | BARTON |

EPO Form 1505.1 03.82

| Category | Citation of document with indication, where appropriate, of relevant passages | Relevant to claim | CLASSIFICATION OF THE APPLICATION (Int Cl 4) |
|---|---|---|---|
| A | AT-B- 387 903 (HRUBY) | | |
| | * Figures 2,7,8; page 4, lines 30-34; page 3, lines 38-39 * | 20, 1,3,4, 8,19 | |
| | -- | | |
| A | EP-A-0 278 993 (DYMAX) | | |
| | * Figure 2; column 3, lines 27-29* | 1,4,7 | |
| | -- | | |
| P,A | US-A-4 883 053 (SIMON) | | TECHNICAL FIELDS SEARCHED (Int Cl 4) |
| | * Column 8, lines 12-14 * | 3,4,8 | |
| | -- | | |
| A | US-A-4 841 967 (CHANG) | | |
| | * Column 7, lines 16-20 * | 1,3, 5-7,19 | |
| | ---- | | |

**DOCUMENTS CONSIDERED TO BE RELEVANT**